# Europäisches Patentamt

# European Patent Office

# Office européen des brevets

(19)

(11) Veröffentlichungsnummer: **0 127 211**
**B1**

(12) **EUROPÄISCHE PATENTSCHRIFT**

(45) Veröffentlichungstag der Patentschrift:
06.08.86

(51) Int. Cl.⁴: **C 07 C 15/46, C 07 C 5/333**

(21) Anmeldenummer: **84200555.5**

(22) Anmeldetag: **18.04.84**

(54) **Verfahren zur Herstellung von Styrol durch Dehydrieren von Äthylbenzol.**

(30) Priorität: **26.05.83 DE 3319024**

(43) Veröffentlichungstag der Anmeldung:
**05.12.84 Patentblatt 84/49**

(45) Bekanntmachung des Hinweises auf die Patenterteilung:
**06.08.86 Patentblatt 86/32**

(84) Benannte Vertragsstaaten:
**DE FR GB IT NL**

(56) Entgegenhaltungen:
**DE - A - 2 909 763**

(73) Patentinhaber: **METALLGESELLSCHAFT AG,
Reuterweg 14 Postfach 3724, D-6000 Frankfurt/M.1 (DE)**
Patentinhaber: **DEGGENDORFER WERFT UND
EISENBAU GMBH, Postfach 1209 Werftstrasse 17,
D-8360 Deggendorf (DE)**

(72) Erfinder: **Möller, Friedrich-Wilhelm, Dr., Breslauer
Ring 15, D-6382 Friedrichsdorf (DE)**
Erfinder: **Buchold, Henning, Dr., Erzbergerstrasse 3,
D-6450 Hanau 1 (DE)**
Erfinder: **Klein, Helmut, Auf dem Unterfeld 2,
D-6450 Hanau am Main (DE)**
Erfinder: **Garkisch, Otto-Ludwig, Dr., Am Dachsbau 4,
D-6232 Bad Soden (DE)**
Erfinder: **Gütlhuber, Friedrich, Gartenstrasse 4,
D-8354 Metten (DE)**
Erfinder: **Laber, Walter, Hengersberger Strasse 61,
D-8360 Deggendorf (DE)**

(74) Vertreter: **Rieger, Harald, Dr., Reuterweg 14,
D-6000 Frankfurt am Main (DE)**

## Beschreibung

Die Erfindung betrifft ein Verfahren zur Dehydrierung von Äthylbenzol im Gemisch mit Wasserdampf zu Styrol an einem in Röhren angeordneten, durch eine Salzschmelze indirekt beheizten Katalysator bei einem Druck von 0,3 bis 1 bar und einer Temperatur der Salzschmelze von 580 bis 660° C, die hochstens 20° C höher ist als die Reaktionstemperatur am Katalysator.

Ein solches Verfahren ist aus dem deutschen Patent 29 09 763 und dem dazu korrespondierenden US-Patent 4 287 375 bekannt. Bei diesem Verfahren wird dem Katalysator ein Gemisch aus Wasserdampf und Äthylbenzol mit einem Gewichtsverhältnis von 1,2 bis 1,5 zugeführt.

Der Erfindung liegt die Aufgabe zugrunde, das bekannte Verfahren weiter zu verbessern, den Verbrauch an Wasserdampf zu senken und gleichzeitig einen hohen Umsatz an Äthylbenzol zu Styrol zu erzielen. Beim eingangs genannten Verfahren wird dies dadurch erreicht, dass der innere Durchmesser der Katalysatorröhren im Bereich von 20 bis 35 mm liegt, den Röhren ein Gemisch aus Wasserdampf und Äthylbenzol mit einem Gewichtsverhältnis von 0,5 bis 1 zugeführt wird und 65 bis 75 Mol-% des Äthylbenzols im Gemisch zu Styrol umgewandelt werden.

Das Verfahren der Erfindung arbeitet mit kommerziellen Katalysatoren, die im wesentlichen aus 50 bis 80% Eisenoxid, 2 bis 10% Chromoxid und Alkali als Rest bestehen.

Die wichtigste Messgrösse ist der Verbrauch an Wasserdampf pro kg gebildetem Styrol, der sich auf diese Weise auf 0,75 bis 1,4 kg einstellt. Trotz des verringerten Dampfeinsatzes arbeitet das Verfahren mit guter Selektivität, da 93 bis 96 Mol-% des am Katalysator umgesetzten Äthylbenzols in Styrol umgewandelt werden, so dass der Rest an Nebenprodukten klein bleibt.

Bei einer bevorzugten Arbeitsweise werden den Katalysatorröhren ein Gemisch aus Wasserdampf und Äthylbenzol mit einem Gewichtsverhältnis von 0,6 bis 0,9 zugeführt. Hierbei liegt der Verbrauch an Wasserdampf pro kg gebildetem Styrol etwa im Bereich von 0,9 bis 1,2 kg. Beim bekannten Verfahren der DE-PS 29 09 763, wo man mit einem Wasserdampf-Äthylbenzol-Gewichtsverhältnis von 1,2 bis 1,5 in dem den Katalysatorröhren zugeführten Gemisch arbeitet, werden pro kg gebildetem Styrol 1,85 bis 2,3 kg Wasserdampf verbraucht.

Es ist zweckmässig, die Salzschmelze zum indirekten Beheizen des Katalysators aus einem Gemisch von Natrium-, Kalium- und Lithiumcarbonat zu bilden. Diese an sich bekannte Salzschmelze vermeidet Korrosionsprobleme und besitzt eine hohe Stabilität.

In der Praxis gibt man das aus Wasserdampf und Äthylbenzol bestehende Einsatzgemisch zur einstufigen isothermen Dehydrierung einem Röhrenreaktor auf, dessen Röhren den Katalysator enthalten, der indirekt durch die heisse Salzschmelze beheizt wird. Das aus dem Röhrenreaktor abgezogene Produkt wird destillativ getrennt, wobei man einen ersten, Styrol und Nebenprodukte enthaltenden Strom abführt, und in einem zweiten Teilstrom nicht umgesetztes Äthylbenzol zum Eingang des Röhrenreaktors zurückleitet. Da die Dehydrierung des Äthylbenzols im Röhrenreaktor sehr temperaturempfindlich ist, muss man, um gute Ausbeuten zu erzielen, die notwendige Reaktionswärme schnell, gleichmässig und möglichst ohne Überhitzungen zuführen. Durch den sehr niedrigen Durchmesser der Katalysatorröhren gelingt dies in optimaler Weise, was zu hervorragenden Ergebnissen des erfindungsgemässen Verfahrens führt.

*Beispiele:*

In einer Versuchsapparatur wird das erfindungsgemässe Verfahren untersucht. In einem Röhrenreaktor mit einem inneren Durchmesser der Katalysatorröhre von 25 mm und einer Rohrlänge von 3 m wird Styrol aus einem Wasserdampf-Äthylbenzol-Gemisch an 1,5 l eines handelsüblichen Katalysators erzeugt. Pro Stunde werden 1,5 l Äthylbenzol über den Katalysator geleitet, der Druck am Reaktor-Austritt beträgt 0,5 bar und das aus einem Gemisch aus Natrium-, Kalium- und Lithiumcarbonat bestehende Salzbad weist eine Temperatur von 620° C auf. Das Einsatzgemisch, jeweils vorgewärmt auf 540° C, wird einmal mit einem Wasserdampf-Äthylbenzol-Gewichtsverhältnis von 0,6 und zum andern von 0,8 über den Katalysator geleitet. In der nachfolgenden Tabelle sind die zugehörigen Ergebnisse dargestellt, dabei ist der Umsatz definiert als der Quotient aus der umgesetzten Menge und der eingesetzten Menge Äthylbenzol, die Selektivität als der Quotient aus der gebildeten Menge Styrol und der umgesetzten Menge Äthylbenzol.

| | 0,6 | 0,8 |
|---|---|---|
| Wasserdampf/Äthylbenzol im Einsatzgemisch (kg/kg) | 0,6 | 0,8 |
| Wasserdampf/erzeugtes Styrol (kg/kg) | 0,89 | 1,1 |
| Umsatz (Mol-%) | 67,5 | 72,8 |
| Selektivität (Mol-%) | 94,9 | 93,8 |
| Das Produkt besteht aus: | | |
| Äthylbenzol (Gew.%) | 32,5 | 27,2 |
| Styrol (Gew.%) | 62,4 | 66,9 |
| Toluol (Gew.%) | 3,4 | 3,9 |
| Benzol (Gew.%) | 1,3 | 1,7 |
| schwersiedender Rest (Gew.%) | 0,4 | 0,3 |

## Patentansprüche

1. Verfahren zur Dehydrierung von Äthylbenzol im Gemisch mit Wasserdampf zu Styrol an einem in Röhren angeordneten, durch eine Salzschmelze indirekt beheizten Katalysator bei einem Druck von 0,3 bis 1 bar und einer Temperatur der Salzschmelze von 580 bis 660° C, die höchstens 20° C höher ist als die Reaktionstemperatur am Katalysator, dadurch gekennzeichnet, dass der innere Durchmesser der Katalysatorröhren im Bereich von 20 bis 35 mm liegt, den Röhren ein·Gemisch aus Wasserdampf und Äthylbenzol mit einem Gewichtsverhältnis von 0,5 bis 1 zugeführt wird und 65 bis 75 Mol % des Äthylbenzols im Gemisch zu Styrol umgewandelt werden.

2. Verfahren nach Anspruch 1, dadurch gekennzeichnet, dass das Gewichtsverhältnis von Wasserdampf im Einsatzgemisch zu gebildetem Styrol 0,75 bis 1,4 beträgt.

3. Verfahren nach Anspruch 1 oder 2, dadurch gekennzeichnet, dass aus 93 bis 96 Mol % des am Katalysator umgesetzten Äthylbenzols Styrol erzeugt wird.

4. Verfahren nach Anspruch 1 oder einem der folgenden, dadurch gekennzeichnet, dass den Katalysatorröhren ein Gemisch aus Wasserdampf und Äthylbenzol mit einem Gewichtsverhältnis von 0,6 bis 0,9 zugeführt wird.

5. Verfahren nach Anspruch 1 oder einem der folgenden, dadurch gekennzeichnet, dass das Gewichtsverhältnis an Wasserdampf im Einsatzgemisch zu gebildetem Styrol 0,9 bis 1,5 beträgt.

## Claims

1. A process of dehydrogenating ethyl benzene in a mixture with steam to form styrene in contact with a catalyst, which is disposed in tubes and indirectly heated by molten salts, which process is carried out under a pressure of 0.3 to 1 bar and with the molten salts at a temperature of 580 to 660° C, which is not more than 20° C in excess of the reaction temperature at the catalyst, characterized in that the inside diameter of the catalyst-containing tubes is between 20 and 35 mm, a mixture of steam and ethyl benzene in a weight ratio of 0.5 to 1 is supplied to the tubes, and 65 to 75 mole percent of the ethyl benzene contained in the mixture are converted to styrene.

2. A process according to claim 1, characterized in that the weight ratio of steam contained in the mixed feedstocks to styrene product is 0.75 to 1.4.

3. A process according to claim 1 or 2, characterized in that 93 to 96 mole percent of the ethyl benzene reacted in contact with the catalyst are converted to styrene.

4. A process according to claim 1 or any of the following claims, characterized in that a mixture of steam and ethyl benzene in a weight ratio of 0.6 to 0.9 is supplied to the catalyst-containing tubes.

5. A process according to claim 1 or any of the following claims, characterized in that the weight ratio of steam contained in the mixed feedstocks to styrene product is 0.9 to 1.5.

## Revendications

1. Procédé de déshydrogénation en styrène d'éthylbenzène en mélange avec de la vapeur d'eau, sur un catalyseur disposé dans des tubes et chauffé indirectement par un sel fondu, en opérant sous une pression de 0,3 à 1 bar et à une température du sel fondu de 580 à 660° C qui est supérieure de 20° C au plus à la température de réaction sur le catalyseur, caractérisé en ce que le diamètre intérieur des tubes pour le catalyseur est de l'ordre de 20 à 35 mm, et le procédé consiste à envoyer dans les tubes un mélange de vapeur d'eau et d'éthylbenzène, en un rapport pondéral de 0,5 à 1, et à transformer en styrène de 65 à 75% en mole de l'éthylbenzène du mélange.

2. Procédé suivant la revendication 1, caractérisé en ce que le rapport pondéral de la vapeur d'eau dans le mélange engagé au styrène formé est compris entre 0,75 et 1,4.

3. Procédé suivant la revendication 1 ou 2, caractérisé en ce qu'il consiste à produire du styrène à partir de 93 à 96% en mole de l'éthylbenzène qui réagit sur le catalyseur.

4. Procédé suivant la revendication 1 ou l'une des revendications suivantes, caractérisé en ce qu'il consiste à envoyer dans les tubes pour le catalyseur un mélange de vapeur d'eau et d'éthylbenzène en un rapport pondéral de 0,6 à 0,9.

5. Procédé suivant la revendication 1 ou l'une des suivantes, caractérisé en ce que le rapport pondéral de la vapeur d'eau dans le mélange engagé au styrène formé est compris entre 0,9 et 1,5.